# EUROPEAN PATENT SPECIFICATION

(11) **EP 3 074 131 B1**
(45) Date of publication and mention of the grant of the patent: **14.11.2018**
(21) Application number: 14843227.1
(22) Date of filing: 25.11.2014
(51) Int. Cl.: B01L 3/00, B01L 3/02, B01L 7/00, C12Q 1/686, G01N 35/10

(54) **TRANSPORTABLE COMPOSITE LIQUID CELLS**
TRANSPORTABLE VERBUNDFLÜSSIGKEITSZELLEN
CELLULES LIQUIDES COMPOSITES TRANSPORTABLES

(30) Priority: 25.11.2013 US 201361908479 P; 25.11.2013 US 201361908489 P
(43) Date of publication of application: 05.10.2016
(73) Proprietor: Gencell Biosystems Ltd., Limerick (IE)
(72) Inventor: BARRETT, Brian, Tipperary (IE); MCGUIRE, David, Limerick (IE); ROEVEN, Robert, Stoughton, WI 53589 (US)
(74) Representative: Duxbury, Stephen
(86) International application number: PCT/IB2014/003058
(87) International publication number: WO 2015/075560

(56) References cited:
- WO-A1-2008/065996
- US-A1- 2012 045 765
- US-A1- 2013 236 377
- US-A1- 2013 260 447

## Description

### BACKGROUND

Current biochemistry processing methods, typically carried out in the macroscopic wells of multi-well plates, has a number of drawbacks that are addressed by the use of composite liquid cells, or CLCs. CLCs are described in U.S. provisional applications Ser. Nos. 61/344,434, filed July 22, 2010, 61/470,515, filed April 1, 2011, and 61/470,520, filed April 1, 2011, and U.S. application no. 13/147,679, filed August 3, 2011. allow for biochemical protocols, such as nucleic acid amplification, to be carried out using far smaller quantities of reagents than would be required for processing in a standard multi-well plate. Use of CLCs also allows for ease of processing, since CLCs can be moved, combined, and divided with relative ease.

An apparatus for performing a reaction in a droplet and a method of using the same, in order to process a biological or chemical sample in the droplet, is disclosed in US 013/0236377 A1. This disclosed apparatus includes a processing compartment, an inlet member with a droplet channel. The description discloses entrapping a droplet of cell media below a contraction in the droplet inlet channel of a chip, from where the droplet is dispensed into the processing compartment and where it mixes with a 3-D matrix which entraps cells in the droplet. A phase of a liquid which is immiscible with the medium in the processing compartment is disposed onto the medium.

### SUMMARY

Devices, systems, and methods for CLCs are disclosed.

### BRIEF DESCRIPTION OF THE DRAWINGS

FIG. 1 schematically shows a cartridge defining a plurality of blind bores.
FIG. 2 schematically shows the cartridge of FIG 1 in cross-section, empty.
FIG. 3 schematically shows the cartridge of FIG 1 in cross-section, with oils and reagents sealed in each blind bore, and includes a close-up of one bore
FIG. 4 schematically shows a plate made of a plurality of cartridges as shown in FIG. 1, shown without seal for clarity.
FIG. 5 schematically shows the plate of FIG. 4 with seals covering the blind bores.
FIG. 6 depicts an embodiment of a system of the invention. Oil A and Oil B are placed into a conduit, which is open at the top and the bottom. Either opening is capable of being closed. The conduit may be in the shape of a conical frustum. Oil B has a higher density than Oil A, and the oils are immiscible. A sample can be added to the conduit. The sample has a density intermediate between the density of Oil A and the density of Oil B. Furthermore, the sample is substantially immiscible with both Oil A and Oil B. The sample, Oil A, and Oil B may be added to the conduit in any order.
FIG. 7 depicts a method of dispensing the sample from the system. Oil B is dispensed from the system through the bottom opening of the conduit. A volume of the sample is dispensed from the system through the bottom opening of the conduit. This may be accomplished by applying positive pressure to the top opening of the conduit. Oil B is returned to the conduit. This may be accomplished by applying negative pressure to the top of the conduit while the bottom of the conduit is contacting Oil B.

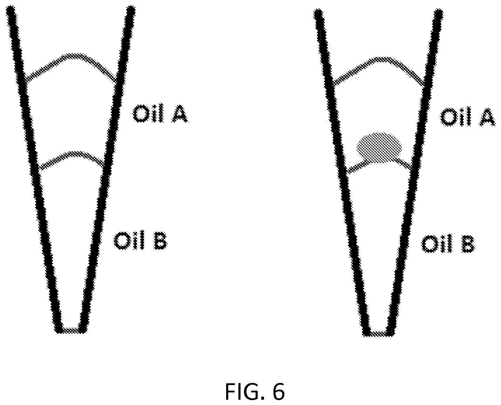

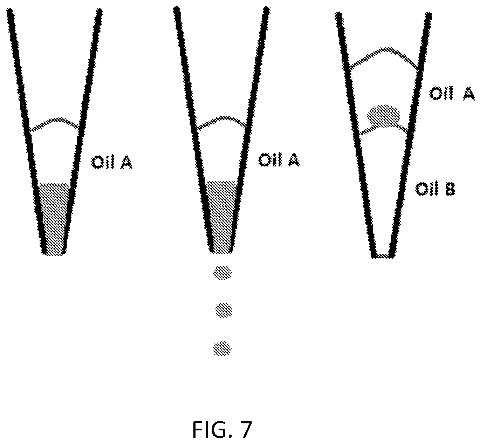

Note that FIGS. 6 and 7 show every meniscus being convex, i.e., bulging upwards toward a central maximum, as would be the case for a typical oil in a polypropylene vessel. If the vessel and/or liquid were differently constituted and had different relative wetting properties, the meniscus could be convex, i.e., bulging downward toward a central minimum, as would be the case for many oils in a vessel having a polytetrafluoroethylene inner surface.

### DETAILED DESCRIPTION

CLCs are generally formed by a combination of three substantially mutually immiscible liquids having three different densities. At the bottom is a carrier fluid, the densest of the three, and at the top is an encapsulating fluid, the least dense. Between the carrier and the encapsulating fluids is the liquid being contained in the CLC, typically aqueous, which can be, for example, a biological sample or a reagent, buffer, or other prescribed element of a biochemical protocol. This general arrangement of three mutually immiscible liquids can be advantageously applied in situations other than manipulation for use in a biochemical protocol, for example, storage in a blind bore in a transportable cartridge, or storage in and dispensing from a device having a through bore, e.g., an openended conduit.

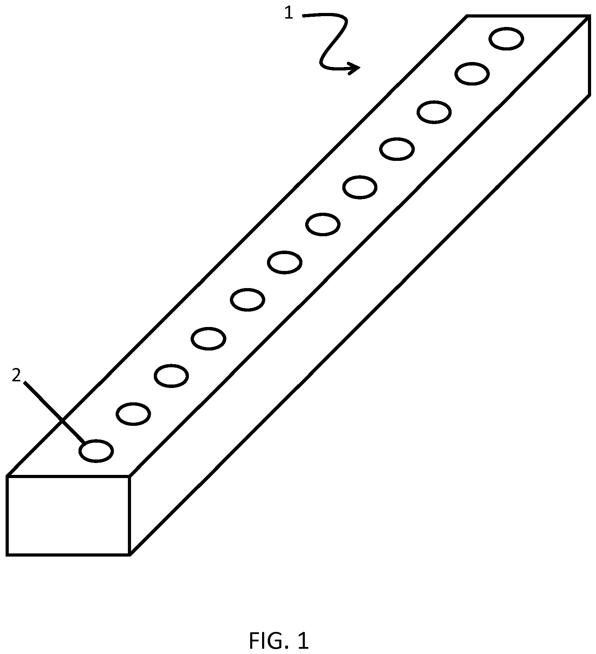

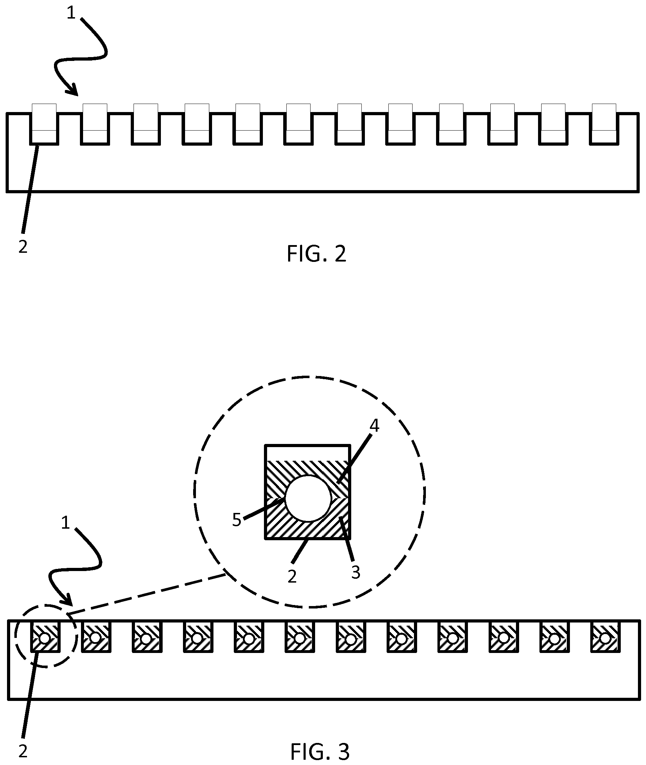

FIG. 1 schematically shows a cartridge 1 defining a plurality of blind bores. The blind bores are co-linear, arranged in a line along the length of the cartridge 1. A first blind bore 2 is positioned at one end of the cartridge 1. FIG. 2 shows the same cartridge 1 in cross-section. As shown, the bores are empty. FIG. 3 shows the same cross-section of the same cartridge **1** as FIG. 2, but in this case the bores are shown filled with fluid. In some embodiments, one or more bores will contain three fluids having three different densities or specific gravities, all three fluids being mutually immiscible. At the bottom of the bore **2** is a high density fluid **3** shown in cross-hatching, for example Fluorinert FC-40 (fluorocarbonated oil) having a density of approximately 1.9 g/cc. Above the high density fluid **3** is a mutually immiscible low density fluid **4** shown in opposite cross-hatching, for example phenylmethylpolysiloxane (silicone oil) having a density of approximately 0.92 g/cc. Between these two oils is an aqueous element **5** having a density of about 1.0 g/cc. Both oils and the aqueous element are all substantially mutually immiscible, giving the contents of each bore many of the functionalities of a CLC.

In some embodiments one or more bores can be left entirely empty, while others include one or more fluids, e.g., oils. In some embodiments, some bores or all the bores may include only the two oils, in anticipation of the addition of an aqueous element. The remaining bores may contain both oils and aqueous elements containing reagents necessary to carry out a biochemical protocol, for example an assay for the presence of a particular bioagent, or the reagents necessary to amplify nucleic acids by PCR. In any case, a sealed cartridge (as discussed below) may be shipped with a CLC-sized quantity of the necessary reagents included in one or more bores. Because the amount of reagent required is so small, the cost of such a cartridge can be kept low. The presence of the two mutually immiscible oils surrounding an aqueous solution also provides stability for the aqueous solution, since the aqueous elements are safely encapsulated in the equivalent of an immobilized CLC.

In some embodiments some or all aqueous components or reagents are dried, e.g., lyophilized or freeze dried, to enhance stability and transport.

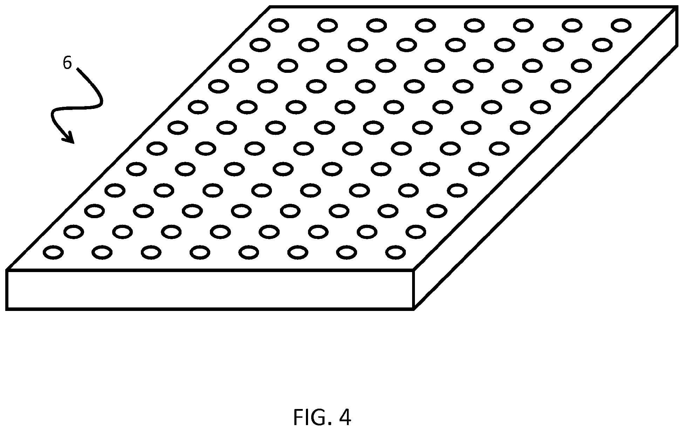

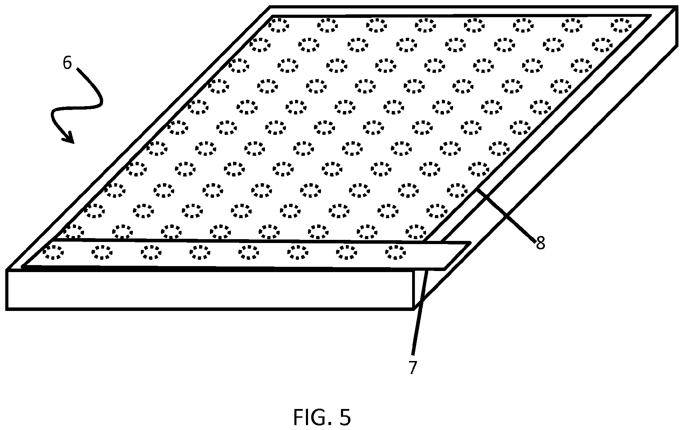

FIGS. 4 and 5 show a series of cartridges, like the ones shown in FIGS. 1-3, assembled together to make a single plate 6. Each row of bores corresponds to a single cartridge. The plate can be made of previously separate cartridges that have been affixed to one another, or the plate can be an integrally formed single piece (in such an embodiment, description herein of the features of a cartridge also apply to a row or section of a unitary or integrally-formed plate). FIG. 5 shows the plate sealed. The first bore in each cartridge is sealed with a tear-away film **7.** In this embodiment, the film **7** is intended to be opened by hand by a user, and samples are to be inserted into the first row of bores, i.e., the first bore in each cartridge. The rest of the bores are covered with a single seal **8.** In this case the seal 8 is a pierceable film. When a cartridge **1** or plate 6 is used, a liquid handling system may access the liquids inside the bores by piercing the seal **8.** The positions of the bores beneath the film **7** and the seal **8** are shown in dotted lines. Although the first bore is shown at one end of the plate/cartridge, it could be positioned anywhere. Any bore could be designated as the bore intended to receive a sample, or be considered the "first" bore. The seal may be any suitable material, e.g., metallic foil or polymeric film or combinations thereof.

A single use cartridge can include a seal and define a plurality of co-linear blind bores, each blind bore defining one opening, at least one of the plurality of blind bores containing two mutually immiscible liquids, both of which are immiscible with water, one of the two mutually immiscible liquids having a specific gravity greater than water and the other of the two mutually immiscible liquids having a specific gravity less than water. The seal can cover the opening defined by the at least one blind bore, thereby making the interior of the blind bore substantially leak proof to the two mutually immiscible liquids.

In some embodiments all the blind bores contain the same two mutually immiscible liquids. In some embodiments at least one blind bore contains only the two mutually immiscible liquids.

In some embodiments the cartridge may have a first blind bore that is (a) positioned at one end of the line of the plurality of blind bores, and (b) is sealed with a portion of the seal adapted to be torn off by a human user by hand thereby exposing the opening of the first blind bore. In some embodiments the first blind bore contains only the two mutually immiscible liquids. In some embodiments at least one of the plurality of blind bores other than the first blind bore contains a reagent that is in an aqueous solution, wherein the reagent is an element of a predetermined biochemical protocol. In some embodiments all the reagents necessary to carry out the predetermined biochemical protocol are separately contained in the plurality of blind bores other than the first blind bore. In some embodiments the predetermined biological protocol is nucleic acid amplification by PCR. In some embodiments at least a portion of the seal is a pierceable film closing the opening defined by at least one blind bore. Optionally, one or more of such reagents are dried, and some bores may be empty to allow for mixing or waste disposal.

In some embodiments the plate can include a plurality of cartridges, the plate being formed by connection or other integration of the plurality of cartridges, the cartridges being arranged in the plate so that each plurality of co-linear blind bores is parallel to all the others. Alternatively, such a plate can be formed with the blind bores in any other pattern, e.g., by use of cartridges having other configurations. As noted, the plate may also be a single, unitary piece.

An embodiment of the system of the invention can include such a cartridge or plate ; a cartridge or plate receiver sized and shaped to mate with the cartridge or plate; a liquid handling system capable of piercing the pierceable film closing any of the plurality of blind bores by inserting a liquid conduit into any of the blind bores when the cartridge has been mated with the cartridge receiver; and a controller operably connected to the liquid handling system. The controller can be programmed to, for example, cause the liquid handling system to: form a composite liquid cell in one of the blind bores; and operate upon the composite liquid cell and the reagents so as to carry out the predetermined biochemical protocol.

In some embodiments the system includes a thermal cycler. In some embodiments the system includes a cooling unit. In some embodiments the system includes optical fluorescence excitation and detection module. In some embodiments the system includes a capillary tube. In some embodiments the system includes a magnetic separation module.

A method of carrying out a biochemical protocol can include providing a system for using a cartridge or plate as described above; inserting the cartridge or plate into the receiver; depositing a biological sample in a first blind bore (either before or after inserting the cartridge or plate into the receiver); activating the controller so that the controller causes the liquid handling system to: form a composite liquid cell that contains an aqueous solution of the biological sample, and operate upon the composite liquid cell and the reagents so as to carry out the predetermined biological protocol on the biological sample.

In addition to systems in which biological samples or reagents are stored in a blind bore, this disclosure also provides in some embodiments systems and methods for the storage and dispensing of a sample from a conduit such as a through bore. The conduit can have flow in either direction and is controlled by a controller. In certain embodiments, a sample, such as a reagent or a biomolecule, is suspended between two fluids, such as oils. In certain embodiments, the sample is used as required and then re-suspended once the required volume of sample is dispensed. In certain embodiments, if a plurality of units of sample is required, the sample may be resuspended after each unit is dispensed, or after a number of units are dispensed, or after all required units of sample are dispensed.

### Exemplary Embodiments

In certain embodiments, the invention relates to a system including a conduit, a pump, a first fluid (e.g., fluid A), a second fluid (e.g., fluid B), and a sample. The conduit can define a top opening and a bottom opening, and can include an internal surface disposed between the top opening and the bottom opening. The first fluid, the sample, and the second fluid can be disposed within the conduit between the top opening and the bottom opening. The first fluid, the second fluid and the sample can be substantially mutually immiscible. The first fluid can be less dense than the sample and the sample can be less dense than the second fluid. The sample can be disposed between the first fluid and the second fluid such that the entire surface area of the sample is in fluid contact with only the first fluid, the second fluid, or the internal surface of the conduit. In some embodiments a pump can configured to apply positive pressure, negative pressure, or no external pressure to a location in the conduit.

In certain embodiments, the invention relates to any one of the systems described herein, wherein the system consists essentially of a conduit such as a through bore, a first fluid (e.g., fluid A), a second fluid (e.g., fluid B), and a sample, and optionally a pump.

In certain embodiments, the invention relates to any one of the systems described herein, wherein the system includes a means for temporarily sealing or temporarily closing the bottom opening of the conduit.

In certain embodiments, the invention relates to any one of the systems described herein, wherein the system includes a means for temporarily sealing or temporarily closing the top opening of the conduit.

In certain embodiments, the invention relates to any one of the systems described herein, wherein the sample is in an aqueous solution.

In certain embodiments, the invention relates to any one of the systems described herein, wherein the sample includes a reagent. Examples of reagents include those useful in sequence bead preparation, pyrosequencing, nucleic acid ligation, and polymerase chain reaction. In certain embodiments, the reagent is on a bead.

In certain embodiments, the invention relates to any one of the systems described herein, wherein the sample includes a biomolecule. Examples of biomolecules include (and are not limited to) cells, nucleic acids, proteins, enzymes, blood, saliva, and organic material. In certain embodiments, the biomolecule is on a bead.

In certain embodiments, the invention relates to any one of the systems described herein, wherein the sample includes more than one reagent or more than one biomolecule, or a reagent and a biomolecule.

In certain embodiments, the invention relates to any one of the systems described herein, wherein the first fluid is immiscible with the second fluid.

In certain embodiments, the invention relates to any one of the systems described herein, wherein the first fluid is immiscible with the sample.

In certain embodiments, the invention relates to any one of the systems described herein, wherein the second fluid is immiscible with the sample.

In certain embodiments, the invention relates to any one of the systems described herein, wherein the first fluid or the second fluid is an oil.

In certain embodiments, the invention relates to any one of the systems described herein, wherein the first fluid is an oil.

In certain embodiments, the invention relates to any one of the systems described herein, wherein the second fluid is an oil.

In certain embodiments, the invention relates to any one of the systems described herein, wherein the oils used for generating immiscible phases, for example the first fluid or the second fluid, can include and are not limited to silicone oil, perfluorocarbon oil, and perfluoropolyether oil.

In certain embodiments, the invention relates to any one of the systems described herein, wherein typical values of densities for the fluids involved range within the values about 1,300 to about 2,000 kg/m³ for the second fluid, about 700 to about 990 kg/m³ for the first fluid, and about 900 to about 1200 kg/m³ for the sample. An example of one such set of operating fluids and densities is outlined herein but is not limited to these; the second fluid is Fluorinert FC-40 (fluorocarbonated oil) density of approximately 1,900 kg/m³; the first fluid is phenylmethylpolysiloxane (silicone oil) density of approximately 920 kg/m³; and the sample is an aqueous based solution of PCR reagents with a density of approximately 1 000 kg/m³.

In certain embodiments, the invention relates to any one of the systems described herein, wherein the second fluid is a perfluorinated amine oil.

In certain embodiments, the invention relates to any one of the systems described herein, wherein the first fluid is a solution of a phenylmethylpolysiloxane-based oil and a polysorbate additive. The additives have a hydrophilic-lipophilic balance number in the range of 2 to 8. The combined total hydrophilic-lipophilic balance number of the additives is in the range of 2 to 8. Examples of polysorbate additives are sorbitane monooleate, sorbitane tristearate, and polysorbate 20 but are not limited to these. These additives within the first fluid range between 0.001% and 10%.

In certain embodiments, the invention relates to any one of the systems described herein, wherein the sample is a solid particle suspension in aqueous media, the first fluid is a phenylmethylpolysiloxane-based oil, and the second fluid is a fluorocarbon-based oil.

In certain embodiments, the invention relates to any one of the systems described herein, wherein the sample is an aqueous media-in-phenylmethylpolysiloxane-based oil, the first fluid is a phenylmethylpolysiloxane-based oil, and the second fluid is a fluorocarbon-based oil.

In certain embodiments, the invention relates to any one of the systems described herein, wherein the conduit is oriented substantially vertically.

In certain embodiments, the invention relates to any one of the systems described herein, wherein the conduit is oriented vertically.

In certain embodiments, the invention relates to any one of the systems described herein, wherein the conduit is a capillary tube.

In certain embodiments, the invention relates to any one of the systems described herein, wherein the conduit is in the shape of a conical frustum.

In certain embodiments, the invention relates to any one of the systems described herein, wherein the conduit is in the shape of a conical frustum; and the top opening of the conduit has an internal diameter that is larger than the internal diameter of the bottom opening of the conduit.

In certain embodiments, the invention relates to any one of the systems described herein, wherein the conduit is a pipette tip.

In certain embodiments, the invention relates to any one of the systems described herein, wherein the conduit is made from a polymer, ceramic, or metal.

In certain embodiments, the invention relates to any one of the systems described herein, wherein the conduit includes a hydrophobic surface.

In certain embodiments, the invention relates to any one of the systems described herein, wherein the conduit is a polymer capillary tube, such as a polytetrafluoroethylene (PTFE) capillary tube.

In certain embodiments, the invention relates to any one of the systems described herein, wherein the conduit is disposable.

In certain embodiments, the invention relates to any one of the systems described herein, wherein the conduit is reusable.

In certain embodiments, the invention relates to any one of the systems described herein, wherein the conduit has an internal diameter is typically within a range of from about 10 microns to about 10 millimetres in diameter.

In certain embodiments, the invention relates to any one of the systems described herein, wherein the internal diameter of the conduit is variable from the bottom of the conduit to the top of the conduit, along the length of the conduit.

In certain embodiments, the invention relates to any one of the systems described herein, wherein the conduit has a wall thickness of at least about 10 microns or more.

In certain embodiments, the invention relates to any one of the systems described herein, wherein the internal shape of the conduit can be (and is not necessarily limited to) a profile which is round, conical, square, oval, rectangular, have a wavy surface, have at least one flat surface, or have surface enhancement features.

In certain embodiments, the invention relates to any one of the systems described herein, wherein the external shape of the conduit can be (and is not necessarily limited to) a profile which is round, conical, square, oval, rectangular, have a wavy surface, have at least one flat surface, or have surface enhancement features.

In certain embodiments, the invention relates to any one of the systems described herein, wherein the pump is a vacuum pump.

In certain embodiments, the invention relates to any one of the systems described herein, wherein the pump is a pipette bulb.

In certain embodiments, the invention relates to any one of the systems described herein, wherein the pump is a means for applying positive pressure.

In certain embodiments, the invention relates to any one of the systems described herein, wherein the pump is a means for applying negative pressure.

In certain embodiments, the invention relates to any one of the systems described herein, wherein the pump is operably connected to the top of the conduit.

In certain embodiments, the invention relates to any one of the systems described herein, wherein the pump is operably connected to the bottom of the conduit.

In certain embodiments, the invention relates to any one of the systems described herein, wherein the system also includes a controller.

In certain embodiments, the invention relates to any one of the systems described herein, wherein the controller is operably connected to the pump.

In certain embodiments, the invention relates to any one of the systems described herein, wherein a plurality of conduits are assembled together in a cassette.

In certain embodiments, the invention relates to any one of the systems described herein, wherein a plurality of conduits are assembled together on a plate.

In certain embodiments, the invention relates to any one of the systems described herein, wherein the system is at about 0°C, about 3°C, about 5°C, about 10 °C, about 15 °C, about 20 °C, about 25 °C, about 30 °C, about 35°C, about 37°C, about 40°C, or about 45°C.

In certain embodiments, the invention relates to any one of the systems described herein, wherein the system is a micropipette.

In certain embodiments, the invention relates to a method for storing a sample in a system, the method including:
providing a conduit, a pump, a first fluid, a second fluid, and a sample, wherein
the conduit has a top opening, a bottom opening, and an internal surface disposed between the top opening and the bottom opening;
the first fluid is substantially immiscible with the second fluid;
the first fluid is substantially immiscible with the sample;
the second fluid is substantially immiscible with the sample;
the first fluid is less dense than the sample;
the sample is less dense than the second fluid; and
the pump is configured to apply positive pressure, negative pressure, or no external pressure to a location in the conduit; and
loading into the conduit, in any order, the first fluid, the second fluid, and the sample, such that the first fluid, the sample, and the second fluid are disposed within the conduit between the top opening and the bottom opening, and the sample is disposed between the first fluid and the second fluid such that the entire surface area of the sample is in fluid contact with only the first fluid, the second fluid, or the internal surface of the conduit.

In certain embodiments, the invention relates to any one of the methods described herein, wherein the system consists essentially of a conduit, a pump, a first fluid (e.g., fluid A), a second fluid (e.g., fluid B), and a sample.

In certain embodiments, the invention relates to any one of the methods described herein, wherein the system includes a means for temporarily sealing or temporarily closing the bottom opening of the conduit.

In certain embodiments, the invention relates to any one of the methods described herein, further including the step of:
temporarily sealing or temporarily closing the bottom opening of the conduit before loading the first fluid, the second fluid, or the sample into the conduit.

In certain embodiments, the invention relates to any one of the methods described herein, wherein the system includes a means for temporarily sealing or temporarily closing the top opening of the conduit.

In certain embodiments, the invention relates to any one of the methods described herein, also including the step of:
temporarily sealing or temporarily closing the top opening of the conduit before loading the first fluid, the second fluid, or the sample into the conduit.

In certain embodiments, the invention relates to any one of the methods described herein, wherein the first fluid is loaded into the conduit before the sample or the second fluid.

In certain embodiments, the invention relates to any one of the methods described herein, wherein the sample is loaded into the conduit before the first fluid or the second fluid.

In certain embodiments, the invention relates to any one of the methods described herein, wherein the second fluid is loaded into the conduit before the first fluid or the sample.

In certain embodiments, the invention relates to any one of the methods described herein, wherein the first fluid is loaded into the conduit via the top opening of the conduit.

In certain embodiments, the invention relates to any one of the methods described herein, wherein the first fluid is loaded into the conduit via the bottom opening of the conduit.

In certain embodiments, the invention relates to any one of the methods described herein, wherein the second fluid is loaded into the conduit via the top opening of the conduit.

In certain embodiments, the invention relates to any one of the methods described herein, wherein the second fluid is loaded into the conduit via the bottom opening of the conduit.

In certain embodiments, the invention relates to any one of the methods described herein, wherein the sample is loaded into the conduit via the top opening of the conduit.

In certain embodiments, the invention relates to any one of the methods described herein, wherein the sample is loaded into the conduit via the bottom opening of the conduit.

In certain embodiments, the invention relates to any one of the methods described herein, wherein the step of loading the first fluid, the second fluid, or the sample into the conduit via the bottom opening of the conduit includes applying negative pressure to the top opening of the conduit.

In certain embodiments, the invention relates to any one of the methods described herein, wherein the sample is in an aqueous solution.

In certain embodiments, the invention relates to any one of the methods described herein, wherein the sample includes a reagent.

In certain embodiments, the invention relates to any one of the methods described herein, wherein the sample includes a biomolecule. Examples of biomolecules include (and are not limited to) cells, nucleic acids, proteins, enzymes, blood, saliva, and organic material.

In certain embodiments, the invention relates to any one of the methods described herein, wherein the first fluid is immiscible with the second fluid.

In certain embodiments, the invention relates to any one of the methods described herein, wherein the first fluid is immiscible with the sample.

In certain embodiments, the invention relates to any one of the methods described herein, wherein the second fluid is immiscible with the sample.

In certain embodiments, the invention relates to any one of the methods described herein, wherein the first fluid or the second fluid is an oil.

In certain embodiments, the invention relates to any one of the methods described herein, wherein the first fluid is an oil.

In certain embodiments, the invention relates to any one of the methods described herein, wherein the second fluid is an oil.

In certain embodiments, the invention relates to any one of the methods described herein, wherein the oils used for generating immiscible phases, for example the first fluid or the second fluid, can include and are not limited to silicone oil, perfluorocarbon oil, and perfluoropolyether oil.

In certain embodiments, the invention relates to any one of the methods described herein, wherein typical values of densities for the fluids involved range within the values about 1,300 to about 2,000 kg/m³ for the second fluid, about 700 to about 990 kg/m³ for the first fluid, and about 900 to about 1200 kg/m³ for the sample. An example of one such set of operating fluids and densities is outlined herein but is not limited to these; the second fluid is Fluorinert FC-40 (fluorocarbonated oil) density of approximately 1,900 kg/m³; the first fluid is phenylmethylpolysiloxane (silicone oil) density of approximately 920 kg/m³; and the sample is an aqueous based solution of PCR reagents with a density of approximately 1000 kg/m³.

In certain embodiments, the invention relates to any one of the methods described herein, wherein the second fluid is a perfluorinated amine oil.

In certain embodiments, the invention relates to any one of the methods described herein, wherein the first fluid is a solution of a phenylmethylpolysiloxane-based oil and a polysorbate additive. The additives have a hydrophilic-lipophilic balance number in the range of 2 to 8. The combined total hydrophilic-lipophilic balance number of the additives is in the range of 2 to 8. Examples of polysorbate additives are sorbitane monooleate, sorbitane tristearate, and polysorbate 20 but are not limited to these. These additives within the first fluid range between 0.001% and 10%.

In certain embodiments, the invention relates to any one of the methods described herein, wherein the sample is a solid particle suspension in aqueous media, the first fluid is a phenylmethylpolysiloxane-based oil, and the second fluid is a fluorocarbon-based oil.

In certain embodiments, the invention relates to any one of the methods described herein, wherein the sample is an aqueous media-in-phenylmethylpolysiloxane-based oil, the first fluid is a phenylmethylpolysiloxane-based oil, and the second fluid is a fluorocarbon-based oil.

In certain embodiments, the invention relates to any one of the methods described herein, wherein the conduit is oriented substantially vertically.

In certain embodiments, the invention relates to any one of the methods described herein, wherein the conduit is oriented vertically.

In certain embodiments, the invention relates to any one of the methods described herein, wherein the conduit is a capillary tube.

In certain embodiments, the invention relates to any one of the methods described herein, wherein the conduit is in the shape of a conical frustum.

In certain embodiments, the invention relates to any one of the methods described herein, wherein the conduit is in the shape of a conical frustum; and the top opening of the conduit has an internal diameter that is larger than the internal diameter of the bottom opening of the conduit.

In certain embodiments, the invention relates to any one of the methods described herein, wherein the conduit is a pipette tip.

In certain embodiments, the invention relates to any one of the methods described herein, wherein the conduit is made from a polymer, ceramic, or metal.

In certain embodiments, the invention relates to any one of the methods described herein, wherein the conduit includes a hydrophobic surface.

In certain embodiments, the invention relates to any one of the methods described herein, wherein the conduit is a polymer capillary tube, such as a PTFE material capillary tube.

In certain embodiments, the invention relates to any one of the methods described herein, wherein the conduit is disposable.

In certain embodiments, the invention relates to any one of the methods described herein, wherein the conduit is reusable.

In certain embodiments, the invention relates to any one of the methods described herein, wherein the conduit has an internal diameter is typically within a range of from about 10 microns to about 10 millimetres in diameter.

In certain embodiments, the invention relates to any one of the methods described herein, wherein the internal diameter of the conduit is variable from the bottom of the conduit to the top of the conduit, along the length of the conduit.

In certain embodiments, the invention relates to any one of the methods described herein, wherein the conduit has a wall thickness of at least about 10 microns or more.

In certain embodiments, the invention relates to any one of the methods described herein, wherein the internal shape of the conduit can be (and is not necessarily limited to) a profile which is round, conical, square, oval, rectangular, have a wavy surface, have at least one flat surface, or have surface enhancement features.

In certain embodiments, the invention relates to any one of the methods described herein, wherein the external shape of the conduit can be (and is not necessarily limited to) a profile which is round, conical, square, oval, rectangular, have a wavy surface, have at least one flat surface, or have surface enhancement features.

In certain embodiments, the invention relates to any one of the methods described herein, wherein the pump is a vacuum pump.

In certain embodiments, the invention relates to any one of the methods described herein, wherein the pump is a pipette bulb.

In certain embodiments, the invention relates to any one of the methods described herein, wherein the pump is a means for applying positive pressure.

In certain embodiments, the invention relates to any one of the methods described herein, wherein the pump is a means for applying negative pressure.

In certain embodiments, the invention relates to any one of the methods described herein, wherein the pump is operably connected to the top of the conduit.

In certain embodiments, the invention relates to any one of the methods described herein, wherein the pump is operably connected to the bottom of the conduit.

In certain embodiments, the invention relates to any one of the methods described herein, wherein the system further includes a controller.

In certain embodiments, the invention relates to any one of the methods described herein, wherein the controller is operably connected to the pump.

In certain embodiments, the invention relates to any one of the methods described herein, wherein a plurality of conduits are assembled together in a cassette.

In certain embodiments, the invention relates to any one of the methods described herein, wherein a plurality of conduits are assembled together on a plate.

In certain embodiments, the invention relates to any one of the methods described herein, also including the step of storing the sample in the system for a period of time.

In certain embodiments, the invention relates to any one of the methods described herein, wherein the first period of time is on the order of minutes, days, months, or years.

In certain embodiments, the invention relates to any one of the methods described herein, also including the step of storing the sample in the system at a temperature of about 0°C, about 3°C, about 5°C, about 10 °C, about 15 °C, about 20 °C, about 25 °C, about 30 °C. about 35°C, about 37°C, about 40°C, or about 45°C.

In certain embodiments, the invention relates to any one of the methods described herein, also including the step of storing the sample in the system for a period of time at a temperature of about 0°C, about 3°C, about 5°C, about 10 °C, about 15 °C, about 20 °C, about 25 °C, about 30 °C, about 35°C, about 37°C, about 40°C, or about 45°C.

In certain embodiments, the invention relates to any one of the methods described herein, wherein the system is on an instrument.

In certain embodiments, the invention relates to any one of the methods described herein, wherein the system is a micropipette.

In certain embodiments, the invention relates to a method of dispensing a sample from any one of the aforementioned systems, including the step of
dispensing from the conduit via the bottom opening the second fluid; and
dispensing from the conduit via the bottom opening a volume of the sample.

In certain embodiments, the invention relates to any one of the methods described herein, also including the step of dispensing from the conduit the first fluid.

In certain embodiments, the invention relates to any one of the methods described herein, wherein the first fluid, the sample, or the second fluid is dispensed from the conduit by negative pressure applied by the pump to the conduit.

In certain embodiments, the invention relates to any one of the methods described herein, wherein the first fluid, the sample, or the second fluid is dispensed from the conduit by positive pressure applied by the pump to the conduit.

In certain embodiments, the invention relates to any one of the methods described herein, wherein the first fluid, the sample, or the second fluid is dispensed from the conduit by negative pressure applied by the pump to the bottom opening of the conduit.

In certain embodiments, the invention relates to any one of the methods described herein, wherein the first fluid, the sample, or the second fluid is dispensed from the conduit by positive pressure applied by the pump to the top opening of the conduit.

In certain embodiments, the invention relates to any one of the methods described herein, also including the step of reloading into the conduit the second fluid.

In certain embodiments, the invention relates to any one of the methods described herein, wherein the second fluid is reloaded into the conduit via the bottom opening.

In certain embodiments, the invention relates to any one of the methods described herein, wherein the second fluid is reloaded into the conduit by negative pressure applied by the pump to the top opening of the conduit.

In certain embodiments, the invention relates to any one of the methods described herein, wherein the second fluid is reloaded into the conduit by positive pressure applied by the pump to the bottom opening of the conduit.

In certain embodiments, the invention relates to any one of the methods described herein, wherein the volume of the sample dispensed from the conduit is from about 10 nL to about 20 µL.

In certain embodiments, the invention relates to any one of the methods described herein, wherein the volume of the sample dispensed from the conduit is about 10 nL, about 20 nL, about 30 nL, about 40 nL, about 50 nL, about 60 nL, about 70 nL, about 80 nL, about 90 nL, about 100 nL, about 200 nL, about 300 nL, about 400 nL, about 500 nL, about 600 nL, about 700 nL, about 800 nL, about 900 nL, 1 µL, about 2 µL, about 3 µL, about 4 µL, about 5 µL, about 6 µL, about 7 µL, about 8 µL, about 9 µL, about 10 µL, about 11 µL, about 12 µL, about 13 µL, about 14 µL, about 15 µL, about 16 µL, about 17 µL, about 18 µL, about 19 µL, or about 20 µL.

In certain embodiments, the invention relates to any one of the methods described herein, wherein the volume of the sample dispensed from the conduit is a predetermined volume.

In certain embodiments, the invention relates to any one of the methods described herein, wherein the system includes a dead volume.

In certain embodiments, the invention relates to any one of the methods described herein, wherein the dead volume is from about 10 nL to about 200 nL.

In certain embodiments, the invention relates to any one of the methods described herein, wherein the dead volume is about 10 nL, about 20 nL, about 30 nL, about 40 nL, about 50 nL, about 60 nL, about 70 nL, about 80 nL, about 90 nL, about 100 nL, about 110 nL, about 120 nL, about 130 nL, about 140 nL, about 150 nL, about 160 nL, about 170 nL, about 180 nL, about 190 nL, or about 200 nL.

In certain embodiments, the invention relates to any one of the methods described herein, wherein the steps are automated.

In certain embodiments, the invention relates to any one of the methods described herein, wherein the steps are automated and controlled by the controller.

In certain embodiments, the invention relates to any one of the methods described herein, also including the step of cleaning or decontaminating the conduit.

In certain embodiments, the invention relates to any one of the methods described herein, wherein the conduit is cleaned or decontaminated with a cleaning agent.

In certain embodiments, the invention relates to any one of the methods described herein, wherein the conduit is reusable; and the cleaning agent is steam.

In certain embodiments, the invention relates to any one of the methods described herein, wherein the conduit is reusable; and the cleaning agent is bleach.

In certain embodiments, the invention relates to any one of the methods described herein, wherein the conduit is reusable; and the cleaning agent includes an enzyme.

In certain embodiments, the invention relates to any one of the methods described herein, wherein the conduit is reusable; and the cleaning agent includes a DNA digestion enzyme.

In certain embodiments, the invention relates to any one of the methods described herein, wherein the cleaning agent includes bleach, a base, a detergent, a surface disinfectant, a mild detergent, a mild acid, or a disinfectant.

Notwithstanding the claims, the invention is also referred to in the following clauses:
1. A single-use cartridge , wherein:
   the cartridge defines a plurality of blind bores, each blind bore defining one opening, at least one of the plurality of blind bores containing two mutually immiscible liquids, both of which are immiscible with water, one of the two mutually immiscible liquids having a specific gravity greater than water and the other of the two mutually immiscible liquids having a specific gravity less than water.
2. The cartridge of clause 1 further comprising a seal that covers the opening defined by the plurality of blind bores, thereby making the interiors of the blind bores substantially leak proof to the two mutually immiscible liquids.
3. The cartridge of clause 2 wherein a first blind bore is sealed with a portion of the seal adapted to be torn off by a human user by hand thereby exposing the opening of the first blind bore.
4. The cartridge of either of clauses 2 or 3 wherein at least a portion of the seal is a pierceable film closing the opening defined by at least one blind bore.
5. The cartridge of any of clauses 1-4 wherein the plurality of blind bores are co-linear.
6. The cartridge of any of clauses 1-5 wherein all the blind bores contain at least the same two mutually immiscible liquids.
7. The cartridge of any of clauses 1-6 wherein at least one blind bore contains only the two mutually immiscible liquids..
8. The cartridge of any of clauses 1-7 wherein a first blind bore contains only the two mutually immiscible liquids.
9. The cartridge of clause 8 wherein at least one of the plurality of blind bores other than the first blind bore contains a reagent that is in an aqueous solution, wherein the reagent is an element of a predetermined biochemical protocol.
10. The cartridge of clause 9 wherein all reagents necessary to carry out the predetermined biochemical protocol are separately contained in the plurality of blind bores other than the first blind bore.
11. The cartridge of clause 10 wherein the predetermined biological protocol is nucleic acid amplification by PCR.
12. A multi-well plate consisting of a plurality of cartridges, each cartridge according to any of clauses 1-11, the plate being integrally formed of the plurality of cartridges, the cartridges being arranged in the plate so that each plurality of co-linear blind bores is parallel to all the others.
13. A system comprising:
   a cartridge according to clause 9;
   a cartridge receiver sized and shaped to mate with the cartridge;
   a liquid handling system capable of piercing the pierceable film closing any of the plurality of blind bores by inserting a liquid conduit into any of the blind bores when the cartridge has been mated with the cartridge receiver; and
   a controller operably connected to the liquid handling system, the controller programmed to cause the liquid handling system to:
   form a composite liquid cell in one of the blind bores; and
   operate upon the composite liquid cell and the reagents so as to carry out the predetermined biochemical protocol.
14. The system of clause 13 further comprising a thermal cycler.
15. The system of clause 13 further comprising a cooling unit.
16. The system of clause 13 further comprising an optical fluorescence excitation and detection module.
17. The system of clause 13 wherein the liquid conduit comprises a capillary tube.
18. The system of clause 13 wherein the liquid conduit comprises a magnetic separation module.
19. A method of carrying out a biochemical protocol comprising:
   providing a system according to clause 13;
   depositing a biological sample in the first blind bore;
   inserting the cartridge into the cartridge receiver;
   activating the controller so that the controller causes the liquid handling system to:
   form a composite liquid cell that contains an aqueous solution of the biological sample; and
   operate upon the composite liquid cell and the reagents so as to carry out the predetermined biological protocol on the biological sample.
20. The method of clause 19 wherein the predetermined biological protocol is designed to carry out one of (a) library preparation, (b) nucleic acid sequencing, (c) PCR, (d) digital PCR, and (e) qPCR.
21. A system comprising a conduit, a pump, a first fluid, a second fluid, and a sample, wherein:
   the conduit defines a top opening and a bottom opening, and includes an internal surface disposed between the top opening and the bottom opening;
   the first fluid, the sample, and the second fluid are disposed within the conduit between the top opening and the bottom opening;
   the first fluid, the second fluid and the sample are substantially mutually immiscible;
   the first fluid is less dense than the sample and the sample is less dense than the second fluid;
   the sample is disposed between the first fluid and the second fluid such that the entire surface area of the sample is in fluid contact with only the first fluid, the second fluid, or the internal surface of the conduit; and
   the pump is configured to apply positive pressure, negative pressure, or no external pressure to a location in the conduit.
22. The system of clause 21, wherein the system further comprises a means for temporarily sealing or temporarily closing the bottom opening of the conduit.
23. The system of clause 21 or 22, wherein the system further comprises a means for temporarily sealing or temporarily closing the top opening of the conduit.
24. The system of any one of clauses 21-23, wherein the sample is in an aqueous solution.
25. The system of any one of clauses 21-24, wherein the sample comprises a reagent.
26. The system of any one of clauses 21-25, wherein the sample comprises a biomolecule.
27. The system of any one of clauses 21-25, wherein the sample comprises a cell, a nucleic acid, a protein, an enzyme, blood, saliva, or an organic material.
28. The system of any one of clauses 21-27, wherein the first fluid is immiscible with the second fluid.
29. The system of any one of clauses 21-28, wherein the first fluid is immiscible with the sample.
30. The system of any one of clauses 21-29, wherein the second fluid is immiscible with the sample.
31. The system of any one of clauses 21-30, wherein the first fluid or the second fluid is an oil.
32. The system of any one of clauses 21-30, wherein the first fluid is an oil.
33. The system of any one of clauses 21-30, wherein the second fluid is an oil.
34. The system of any one of clauses 21-30, wherein the first fluid or the second fluid is a silicone oil, a perfluorocarbon oil, or a perfluoropolyether oil.
35. The system of any one of clauses 21-34, wherein the density of the second fluid is from about 1,300 to about 2,000 kg/m3.
36. The system of any one of clauses 21-35, wherein the density of the first fluid is from about 700 to about 990 kg/m3.
37. The system of any one of clauses 21-36, wherein the density of the sample is from about 900 to about 1200 kg/m3.
38. The system of any one of clauses 21-37, wherein the second fluid is a fluorocarbonated oil with a density of about 1,900 kg/m3.
39. The system of any one of clauses 21-38, wherein the first fluid is phenylmethylpolysiloxane.
40. The system of any one of clauses 21-39, wherein the sample is an aqueous based solution of PCR reagents with a density of approximately 1000 kg/m3.
41. The system of any one of clauses 21-37, wherein the second fluid is a perfluorinated amine oil.
42. The system of any one of clauses 21-38, wherein the first fluid is a solution of a phenylmethylpolysiloxane-based oil and a polysorbate additive.
43. The system of any one of clauses 21-37, wherein the sample is a solid particle suspension in aqueous media, the first fluid is a phenylmethylpolysiloxane-based oil, and the second fluid is a fluorocarbon-based oil.
44. The system of any one of clauses 21-37, wherein the sample is an aqueous media-in-phenylmethylpolysiloxane-based oil, the first fluid is a phenylmethylpolysiloxane-based oil, and the second fluid is a fluorocarbon-based oil.
45. The system of any one of clauses 21-44, wherein the conduit is oriented substantially vertically.
46. The system of any one of clauses 21-44, wherein the conduit is oriented vertically.
47. The system of any one of clauses 21-46, wherein the conduit is a capillary tube.
48. The system of any one of clauses 21-46, wherein the conduit is in the shape of a conical frustum.
49. The system of any one of clauses 21-46, wherein the conduit is in the shape of a conical frustum; and the top opening of the conduit has an internal diameter that is larger than the internal diameter of the bottom opening of the conduit.
50. The system of any one of clauses 21-46, wherein the conduit is a pipette tip.
51. The system of any one of clauses 21-50, wherein the conduit is made from a polymer, ceramic, or metal.
52. The system of any one of clauses 21-51, wherein the conduit comprises a hydrophobic surface.
53. The system of any one of clauses 21-50, wherein the conduit is a polymer capillary tube, such as a polytetrafluoroethylene (PTFE) capillary tube.
54. The system of any one of clauses 21-53, wherein the conduit is disposable.
55. The system of any one of clauses 21-53, wherein the conduit is reusable.
56. The system of any one of clauses 21-55, wherein the pump is a vacuum pump.
57. The system of any one of clauses 21-55, wherein the pump is a pipette bulb.
58. The system of any one of clauses 21-55, wherein the pump is a means for applying positive pressure.
59. The system of any one of clauses 21-55, wherein the pump is a means for applying negative pressure.
60. The system of any one of clauses 21-55, wherein the pump is operably connected to the top opening of the conduit.
61. The system of any one of clauses 21-55, wherein the pump is operably connected to the bottom of the conduit.
62. The system of any one of clauses 21-61, wherein the system further comprises a controller.
63. The system of clause 62, wherein the controller is operably connected to the pump.
64. The system of any one of clauses 21-63, wherein a plurality of conduits is assembled together on a plate.
65. A method of dispensing the sample from the system of any one of clauses 21-64, comprising the step of
   dispensing from the conduit via the bottom opening the second fluid; and
   dispensing from the conduit via the bottom opening a volume of the sample.
66. The method of clause 65, further comprising the step of dispensing from the conduit the first fluid.
67. The method of clause 65 or 66, wherein the first fluid, the sample, or the second fluid is dispensed from the conduit by negative pressure applied by the pump to the conduit.
68. The method of clause 65 or 66, wherein the first fluid, the sample, or the second fluid is dispensed from the conduit by positive pressure applied by the pump to the conduit.
69. The method of clause 65 or 66, wherein the first fluid, the sample, or the second fluid is dispensed from the conduit by negative pressure applied by the pump to the bottom opening of the conduit.
70. The method of clause 65 or 66, wherein the first fluid, the sample, or the second fluid is dispensed from the conduit by positive pressure applied by the pump to the top opening of the conduit.
71. The method of any one of clauses 45-70, further comprising the step of reloading into the conduit the second fluid.
72. The method of clause 71, wherein the second fluid is reloaded into the conduit via the bottom opening.
73. The method of clause 71, wherein the second fluid is reloaded into the conduit by negative pressure applied by the pump to the top opening of the conduit.
74. The method of clause 71, wherein the second fluid is reloaded into the conduit by positive pressure applied by the pump to the bottom opening of the conduit.
75. The method of any one of clauses 65-74, wherein the volume of the sample dispensed from the conduit is from about 10 nL to about 20 µL.
76. The method of any one of clauses 65-75, wherein the volume of the sample dispensed from the conduit is a predetermined volume.
77. The method of any one of clauses 65-76, wherein the steps are automated.
78. The method of any one of clauses 65-76, wherein the steps are automated and controlled by the controller.
79. A method for storing a sample in a system, the method comprising:
   providing a conduit, a pump, a first fluid, a second fluid, and a sample, wherein
   the conduit defines a top opening and a bottom opening, and includes an internal surface disposed between the top opening and the bottom opening;
   the first fluid, the second fluid and the sample are substantially mutually immiscible;
   the first fluid is less dense than the sample and the sample is less dense than the second fluid; and
   the pump is configured to apply positive pressure, negative pressure, or no external pressure to a location in the conduit; and
   loading into the conduit, in any order, the first fluid, the second fluid, and the sample, such that the first fluid, the sample, and the second fluid are disposed within the conduit between the top opening and the bottom opening, and the sample is disposed between the first fluid and the second fluid such that the entire surface area of the sample is in fluid contact with only the first fluid, the second fluid, or the internal surface of the conduit.
80. The method of clause 79, wherein the system is a system of any one of clauses 21-64.
81. The method of clause 79 or 80, further comprising the step of:
   temporarily sealing or temporarily closing the bottom opening of the conduit before loading the first fluid, the second fluid, or the sample into the conduit.
82. The method of any one of clauses 79-81, further comprising the step of:
   temporarily sealing or temporarily closing the top opening of the conduit before loading the first fluid, the second fluid, or the sample into the conduit.
83. The method of any one of clauses 79-82, wherein the first fluid is loaded into the conduit before the sample or the second fluid.
84. The method of any one of clauses 79-82, wherein the sample is loaded into the conduit before the first fluid or the second fluid.
85. The method of any one of clauses 79-82, wherein the second fluid is loaded into the conduit before the first fluid or the sample.
86. The method of any one of clauses 79-85, wherein the first fluid is loaded into the conduit via the top opening of the conduit.
87. The method of any one of clauses 79-85, wherein the first fluid is loaded into the conduit via the bottom opening of the conduit.
88. The method of any one of clauses 79-87, wherein the second fluid is loaded into the conduit via the top opening of the conduit.
89. The method of any one of clauses 79-87, wherein the second fluid is loaded into the conduit via the bottom opening of the conduit.
90. The method of any one of clauses 79-89, wherein the sample is loaded into the conduit via the top opening of the conduit.
91. The method of any one of clauses 79-89, wherein the sample is loaded into the conduit via the bottom opening of the conduit.
92. The method of any one of clauses 79-85, wherein the step of loading the first fluid, the second fluid, or the sample into the conduit via the bottom opening of the conduit comprises applying negative pressure to the top opening of the conduit.

### Applications

### Composite Liquid Cell Processing

In one embodiment, the sample is dispensed into an immiscible fluid cell positioned on a free surface of a mutually immiscible fluid. The resulting composite fluid cell can be transported, and/or merged, and/or mixed, and/or have biochemical processing performed on it.

In one embodiment, the sample is dispensed into an immiscible fluid cell positioned on a free surface of a mutually immiscible fluid with a mechanical stabilization feature.

In one embodiment, the upon dispensing from the conduit onto a free surface, the second fluid, the sample, and the first fluid generate a composite liquid cell.

In one embodiment, the sample has paramagnetic beads and buffer.

In one embodiment, the sample contains a buffer.

Examples of composite liquid cell systems to which the present systems and methods can be adapted are disclosed, for example, in PCT/IE2011/000040.

### Sequencing

Many next generation sequencing (NGS) platforms require DNA libraries made up of DNA fragments within a specific range of base pair lengths. In addition, these DNA fragments need to be tagged with specific nucleotide sequences (adapters) to allow the sequences to be amplified using PCR and to allow the library fragments to anneal to the sequencer flow cell. Sequence specific indices can also be added to the DNA fragments to identify individual samples when multiplexing sample within a single flow cell. The tagmentation of DNA (DNA is fragmented and tagged with adapters) and the addition of common adapters and indices is achieved in two separate biological reactions. Following these reactions, the DNA library is cleaned to remove excess nucleotides, enzymes, primers, salts and other contaminants. Consequently, the workflow required to tagment DNA, purify tagmented DNA, add common adapters and indices and purify the final library product is complex and labour intensive. In one embodiment the aforementioned systems and methods can be used to automate genetic sequencing.

### Genetic Sequencing Bead Coating

Genetic sequencing bead preparation is a process by which small beads are coated in an application-specific chemistry. In one embodiment the coating of beads in advance of genetic screening is achieved by the systems and methods of the invention.

These methods provide for a convenient way of manipulating and combining sub-microlitre volumes of fluid that is currently not possible to achieve using conventional techniques, thereby reducing the initial sample volumes and improving the bead coating efficiency by reducing the reaction volume. Further processing using PCR and thermal cycling and genetic sequencing is application-specific.

The use of this technology greatly simplifies the collection procedure for these relatively small target volumes. The system facilitates 100% volume retrieval as the biological sample in processing does not incur any pipetting loses. These features make automation of the biochemistry process easier to facilitate.

### Size Selection of DNA libraries for NGS sequencing

Each of the next generation sequencers have an optimal read length (base pairs). During library construction, DNA is fragmented into DNA molecules with a wide base pair length range. Size selection is currently performed using paramagnetic beads on a microtitre plate and is labour intensive and suffers from inefficiencies from pipetting errors and user protocol variations. The systems and methods of the invention can be used for size selection of DNA libraries.

### Nucleic Acid Purification

The systems and methods of the invention can be used for purification and/or isolation of samples before and/or after PCR.

### Genotyping

The systems and methods of the invention can be used for the storage and dispensing of genotyping reagents or samples.

### Compound Screening

The systems and methods of the invention can be used for the storage and dispensing of drug compound screening reagents or samples.

### EXAMPLES

The following examples illustrate particular embodiments, but should not be viewed as limiting the scope of the disclosed subject matter.

### Example 1

A number of chambers or wells, for example from 1 to 384 chambers, are filled with two immiscible oils such that one oil sits on top of the other (see FIG. 1). Each chamber defines an opening in the bottom. The chambers may be arrayed on a store plate. Reagent is added to the chambers and lies at the interface between the two oils, forming what in some cases will be a Composite Liquid Cell, or CLC (Composite Liquid Cells are described in detail in U.S. Patent No. 8,465,707. In this way, the reagent can be stored at room temperature for long periods of time with no degradation to the reagent from freezing and/or thawing. As and when required the lower oil, oil B, is removed by positively pressurizing the chamber above oil A and forcing the lower oil out of the bottom of the chamber. The reagent then moves down to the orifice and is ready to be dispensed (see FIG. 2). Using the pressure dispensing method, droplets can be created and dispensed directly from the store plate in parallel from the plurality of chambers in the store plate. Droplets can be dispensed into another CLC, well plate, or to any chamber or fluid as required. Once the reagent has finished dispensing it can be returned to storage by drawing an aliquot of oil B and again suspending the reagent as a droplet between Oil B and Oil A. There are no issues with contamination as it is the same reagent used during the entire process and therefore can be repeated any number of times until the reagent runs out. The plate can be loaded at any time, discarded, or cleaned and reused.

## Claims

1. A single-use cartridge (1), wherein:
the cartridge (1) defines a plurality of blind bores (2), each blind bore defining one opening, at least one of the plurality of blind bores (2) containing two mutually immiscible liquids (3,4) both of which are immiscible with water, one of the two mutually immiscible liquids (3,4) having a specific gravity greater than water and the other of the two mutually immiscible liquids having a specific gravity less than water.

2. The cartridge (1) of claim 1, further comprising a seal that (8) covers the opening defined by the plurality of blind bores (2), thereby making the interiors of the blind bores (2) leak proof to the two mutually immiscible liquids (3,4), preferably wherein a first blind bore (2) is sealed with a portion of the seal (8) adapted to be torn off by a human user by hand thereby exposing the opening of the first blind bore (2) .

3. The cartridge (1) of claim 2, wherein at least a portion of the seal (8) is a pierceable film closing the opening defined by at least one blind bore (2) .

4. The cartridge (1) of any of claims 1-3, with one or more of the following:
• wherein the plurality of blind bores (2) are co-linear,
• wherein all the blind bores (2) contain at least the same two mutually immiscible liquids (3,4),
• at least one blind bore contains only the two mutually immiscible liquids (3,4),
• wherein a first blind bore contains only the two mutually immiscible liquids (3,4) .

5. The cartridge (1) of claim 4, wherein at least one of the plurality of blind bores (2) other than the first blind bore contains a reagent that is in an aqueous solution, wherein the reagent is an element of a predetermined biochemical protocol.

6. The cartridge (1) of claim 5, wherein all reagents necessary to carry out the predetermined biochemical protocol are separately contained in the plurality of blind bores (2) other than the first blind bore (2).

7. The cartridge (1) of claim 6, wherein the predetermined biological protocol is nucleic acid amplification by PCR.

8. A multi-well plate (6) consisting of a plurality of cartridges (1), each cartridge (1) according to any of claims 1-7, the plate (6) being integrally formed of the plurality of cartridges (1), the cartridges (1) being arranged in the plate (6) so that each plurality of co-linear blind bores (2) is parallel to all the others.

9. A system comprising:
a cartridge (1) according to claim 5,
a cartridge receiver sized and shaped to mate with the cartridge (1);
a liquid handling system capable of piercing the pierceable film closing any of the plurality of blind bores (2) by inserting a liquid conduit into any of the blind bores (2) when the cartridge (1) has been mated with the cartridge receiver; and
a controller operably connected to the liquid handling system, the controller programmed to cause the liquid handling system to:
form a composite liquid cell in one of the blind bores (2); and
operate upon the composite liquid cell and the reagents so as to carry out the predetermined biochemical protocol.

10. The system of claim 9, further comprising one of the following:
• a thermal cycler,
• a cooling unit,
• an optical fluorescence excitation and detection module,
• wherein the liquid conduit comprises a capillary tube,
• wherein the liquid conduit comprises a magnetic separation module.

11. A method of carrying out a biochemical protocol comprising:
providing a system according to claim 9,
depositing a biological sample in the first blind bore;
inserting the cartridge (1) into the cartridge receiver;
activating the controller so that the controller causes the liquid handling system to:
form a composite liquid cell that contains an aqueous solution of the biological sample; and
operate upon the composite liquid cell and the reagents so as to carry out the predetermined biological protocol on the biological sample.

12. The method of claim 11, wherein the predetermined biological protocol is designed to carry out one of (a) library preparation, (b) nucleic acid sequencing, (c) PCR, (d) digital PCR, and (e) qPCR.

13. A system comprising a a conduit, a pump, a first fluid, a second fluid, and a sample, wherein:
the conduit defines a top opening and a bottom opening, and includes an internal surface disposed between the top opening and the bottom opening;
the first fluid, the sample, and the second fluid are disposed within the conduit between the top opening and the bottom opening;
the first fluid, the second fluid and the sample are mutually immiscible;
the first fluid is less dense than the sample and the sample is less dense than the second fluid;
the sample is disposed between the first fluid and the second fluid such that the entire surface area of the sample is in fluid contact with only the first fluid, the second fluid, or the internal surface of the conduit;
the pump is configured to apply positive pressure, negative pressure, or no external pressure to a location in the conduit, and
a cartridge according to any of the preceding claims 1-7.

14. A method of dispensing the sample from the system of claim 13 comprising the step of
dispensing from the conduit via the bottom opening the second fluid; and
dispensing from the conduit via the bottom opening a volume of the sample.

15. A method for storing a sample in a system, the method comprising:
providing a cartridge (1) according to any of the preceding claims 1-7, a conduit, a pump, a first fluid, a second fluid, and a sample, wherein
the cartridge (1) defines a top opening and a bottom opening, and includes an internal surface disposed between the top opening and the bottom opening;
the first fluid, the second fluid and the sample are mutually immiscible; the first fluid is less dense than the sample and the sample is less dense than the second fluid; and
the pump is configured to apply positive pressure, negative pressure, or no external pressure to a location in the conduit; and
loading into the conduit, in any order, the first fluid, the second fluid, and the sample, such that the first fluid, the sample, and the second fluid are disposed within the conduit between the top opening and the bottom opening, and the sample is disposed between the first fluid and the second fluid such that the entire surface area of the sample is in fluid contact with only the first fluid, the second fluid, or the internal surface of the conduit.

## Patentansprüche

1. Eine Einwegkartusche (1), wobei:
die Kartusche (1) mehrere Blindbohrungen (2) bildet, wobei jede Blindbohrung eine Öffnung bildet, mindestens eine der mehreren Blindbohrungen (2) zwei miteinander nicht mischbare Flüssigkeiten (3, 4) enthält, die beide nicht mischbar mit Wasser sind, eine der zwei miteinander nicht mischbaren Flüssigkeiten (3, 4) eine höhere spezifische Dichte als Wasser und die andere der beiden miteinander nicht mischbaren Flüssigkeiten eine spezifische Dichte geringer als Wasser aufweisen.

2. Die Kartusche (1) nach Anspruch 1, weiterhin umfassend eine Dichtung (8), welche die Öffnung, die durch die mehreren Blindbohrungen (2) gebildet wird, bedeckt, wodurch die Innenräume der Blindbohrungen (2) auslaufsicher für die beiden miteinander nicht mischbaren Flüssigkeiten (3, 4) gemacht werden, wobei bevorzugt eine erste Blindbohrung (2) mit einem Teil der Dichtung (8) abgedichtet ist, welcher angepasst ist, um von einem humanen Benutzer per Hand abgerissen zu werden, wodurch die Öffnung der ersten Blindbohrung (2) freigelegt wird.

3. Die Kartusche (1) nach Anspruch 2, wobei mindestens ein Teil der Dichtung (8) ein durchstechbarer Film ist, der die von mindestens einer Blindbohrung (2) gebildete Öffnung verschließt.

4. Die Kartusche (1) nach einem der Ansprüche 1 bis 3, mit einem oder mehreren der folgenden:
wobei die Mehrzahl der Blindbohrungen (2) kollinear sind,
wobei alle Blindbohrungen (2) mindestens die beiden gleichen, miteinander nicht mischbaren Flüssigkeiten (3, 4) enthalten,
mindestens eine Blindbohrung nur die beiden miteinander nicht mischbaren Flüssigkeiten (3, 4) enthält,
wobei eine erste Blindbohrung nur die beiden miteinander nicht mischbaren Flüssigkeiten (3, 4) enthält.

5. Die Kartusche (1) nach Anspruch 4, wobei mindestens eine andere der mehreren Blindbohrungen (2) als die erste Blindbohrung ein Reagens enthält, welches eine wässrige Lösung ist, wobei das Reagens ein Element eines vorbestimmten biochemischen Protokolls ist.

6. Die Kartusche (1) nach Anspruch 5, wobei alle Reagenzien, welche notwendig sind, das vorbestimmte biochemische Protokoll durchzuführen, separat in den mehreren anderen Blindbohrungen (2) als der ersten Blindbohrung (2) enthalten sind.

7. Die Kartusche (1) nach Anspruch 6, wobei das vorbestimmte biochemische Protokoll eine Nukleinsäure-Amplifizierung durch PCR ist.

8. Eine Multi-Well-Platte (6), bestehend aus einer Mehrzahl von Kartuschen (1), jede Kartusche (1) gemäß einem der Ansprüche 1 bis 7, wobei die Platte (6) einstückig von der Mehrzahl der Kartuschen (1) ausgebildet wird und die Kartuschen (1) so in der Platte (6) angeordnet sind, dass jede Mehrzahl von kollinearen Blindbohrungen (2) parallel zu allen anderen ist.

9. Ein System, umfassend:
eine Kartusche (1) gemäß Anspruch 5,
eine Kartuschenaufnahme, welche so dimensioniert und ausgeformt ist, dass sie mit der Kartusche (1) zusammenpasst;
ein System zur Handhabung von Flüssigkeiten, welches in der Lage ist, den durchstechbaren Film, welcher irgendeines der Mehrzahl von Blindbohrungen (2) verschließt, durch Einführung einer Flüssigkeitsleitung in irgendeine der Blindbohrungen (2) zu durchstechen, wenn die Kartusche (1) mit der Kartuschenaufnahme zusammengefügt wurde; und
eine Steuereinheit, welche funktional mit dem System zur Handhabung von Flüssigkeiten verbunden ist, wobei die Steuereinheit programmiert ist, um zu bewirken, dass das System zur Handhabung von Flüssigkeiten:
eine Komposit-Flüssigkeitszelle in einer der Blindbohrungen (2) bildet; und
so auf die Komposit-Flüssigkeitszelle und die Reagenzien einwirkt, dass das vorbestimmte biochemische Protokoll durchgeführt wird.

10. Das System nach Anspruch 9, weiterhin umfassend eines der folgenden:
• einen Thermalzykler,
• eine Kühleinheit,
• ein optisches Fluoreszenzanregungs- und -detektionsmodul,
• wobei die Flüssigkeitsleitung eine Kapillarröhre umfasst,
• wobei die Flüssigkeitsleitung ein magnetisches Separationsmodul umfasst.

11. Ein Verfahren zur Durchführung eines biochemischen Protokolls, umfassend:
Bereitstellung eines Systems gemäß Anspruch 9;
Ablagern einer biologischen Probe in der ersten Blindbohrung;
Inserieren der Kartusche (1) in die Kartuschenaufnahme;
Aktivieren der Steuereinheit, so dass die Steuereinheit das Flüssigkeitshandhabungssystem dazu bewegt:
eine Komposit-Flüssigkeitszelle, welche eine flüssige Lösung der biologischen Probe enthält zu bilden; und
so auf die Komposit-Flüssigkeitszelle und die Reagenzien einzuwirken, dass das vorbestimmte biologische Protokoll an der biologischen Probe ausgeführt wird.

12. Ein Verfahren nach Anspruch 11, wobei das vorbestimmte biologische Protokoll ausgestaltet ist, um eines der Verfahren (a) Bibliothekherstellung (b) Nukleinsäuresequenzierung, (c) PCR, (d) digitale PCR und (e) qPCR durchzuführen.

13. Ein System, umfassend eine Leitung, eine Pumpe, eine erste Flüssigkeit, eine zweite Flüssigkeit und eine Probe, wobei:
die Leitung eine obere und eine untere Öffnung ausbildet und eine innere Oberfläche umfasst, welche zwischen der oberen Öffnung und der unteren Öffnung angeordnet ist;
die erste Flüssigkeit, die Probe und die zweite Flüssigkeit innerhalb der Leitung zwischen der oberen Öffnung und der unteren Öffnung angeordnet sind;
die erste Flüssigkeit, die zweite Flüssigkeit und die Probe miteinander nicht mischbar sind;
die erste Flüssigkeit weniger dicht als die Probe und die Probe weniger dicht als die zweite Flüssigkeit ist;
die Probe zwischen der ersten Flüssigkeit und der zweiten Flüssigkeit so angeordnet ist, dass die gesamte Oberfläche der Probe nur mit der ersten Flüssigkeit, der zweiten Flüssigkeit oder der inneren Oberfläche der Leitung in flüssigem Kontakt steht;
die Pumpe eingerichtet ist, um positiven Druck, negativen Druck oder keinen externen Druck auf eine Stelle in der Leitung auszuüben, und
eine Kartusche gemäß einem der vorhergehenden Ansprüche 1 bis 7.

14. Das Verfahren zur Dispensierung einer Probe aus dem System nach Anspruch 13, umfassend die Schritte:
Dispensieren der zweiten Flüssigkeit aus der Leitung über die untere Öffnung; und
Dispensieren eines Volumens der Probe aus der Leitung über die untere Öffnung.

15. Ein Verfahren zur Lagerung einer Probe in einem System, wobei das Verfahren umfasst:
Bereitstellen einer Kartusche (1) gemäß einem der vorhergehenden Ansprüche 1 bis 7, einer Leitung, einer Pumpe, einer ersten Flüssigkeit, einer zweiten Flüssigkeit und einer Probe, wobei
die Kartusche (1) eine obere und eine untere Öffnung bildet und eine innere Oberfläche aufweist, welche zwischen der oberen Öffnung und der unteren Öffnung angeordnet ist;
die erste Flüssigkeit, die zweite Flüssigkeit und die Probe miteinander nicht mischbar sind;
die erste Flüssigkeit eine geringere Dichte als die Probe besitzt und die Probe eine geringere Dichte als die zweite Flüssigkeit; und
die Pumpe eingerichtet ist, um einen positiven Druck, negativen Druck oder keinen externen Druck auf eine Stelle in der Leitung auszuüben; und
Beladen der Leitung mit der ersten Flüssigkeit, der zweiten Flüssigkeit und der Probe in einer beliebigen Reihenfolge, so dass die erste Flüssigkeit, die Probe und die zweite Flüssigkeit innerhalb der Leitung zwischen der oberen Öffnung und der unteren Öffnung angeordnet sind, und die Probe zwischen der ersten Flüssigkeit und der zweiten Flüssigkeit so angeordnet ist, dass die gesamte Oberfläche der Probe nur mit der ersten Flüssigkeit, der zweiten Flüssigkeit oder der inneren Oberfläche der Leitung in flüssigem Kontakt steht.

## Revendications

1. Cartouche à usage unique (1), dans laquelle :
la cartouche (1) définit une pluralité d'alésages borgnes (2), chaque alésage borgne définissant une ouverture, au moins l'un de la pluralité d'alésages borgnes (2) contenant deux liquides mutuellement non miscibles (3, 4) dont les deux sont non miscibles avec l'eau, l'un des deux liquides mutuellement non miscibles (3, 4) ayant une densité supérieure à celle de l'eau et l'autre des deux liquides mutuellement non miscibles ayant une densité inférieure à celle de l'eau.

2. Cartouche (1) selon la revendication 1, comprenant en outre un joint d'étanchéité (8) qui couvre l'ouverture définie par la pluralité d'alésages borgnes (2), rendant ainsi les intérieurs des alésages borgnes (2) étanches aux deux liquides mutuellement non miscibles (3, 4), de préférence dans laquelle un premier alésage borgne (2) est étanchéifié avec une portion du joint d'étanchéité (8) adaptée pour être déchirée à la main par un utilisateur humain exposant ainsi l'ouverture du premier alésage borgne (2).

3. Cartouche (1) selon la revendication 2, dans laquelle au moins une portion du joint d'étanchéité (8) est un film perçable fermant l'ouverture définie par au moins un alésage borgne (2).

4. Cartouche (1) selon l'une quelconque des revendications 1 à 3, avec un ou plusieurs de ce qui suit :
- dans laquelle la pluralité d'alésages borgnes (2) sont colinéaires,
- dans laquelle tous les alésages borgnes (2) contiennent au moins deux liquides mutuellement non miscibles identiques (3, 4),
- au moins un alésage borgne contient uniquement les deux liquides mutuellement non miscibles (3, 4),
- dans laquelle un premier alésage borgne contient uniquement les deux liquides mutuellement non miscibles (3, 4).

5. Cartouche (1) selon la revendication 4, dans laquelle au moins l'un de la pluralité d'alésages borgnes (2) autre que le premier alésage borgne contient un réactif qui est dans une solution aqueuse, dans laquelle le réactif est un élément d'un protocole biochimique prédéterminé.

6. Cartouche (1) selon la revendication 5, dans laquelle tous les réactifs nécessaires pour réaliser le protocole biochimique prédéterminé sont contenus séparément dans la pluralité d'alésages borgnes (2) autres que le premier alésage borgne (2).

7. Cartouche (1) selon la revendication 6, dans laquelle le protocole biologique prédéterminé est une amplification d'acide nucléique par PCR.

8. Plaque multipuits (6) constituée par une pluralité de cartouches (1), chaque cartouche (1) étant selon l'une quelconque des revendications 1 à 7, la plaque (6) étant formée d'un seul tenant de la pluralité de cartouches (1), les cartouches (1) étant agencées dans la plaque (6) de sorte que chaque pluralité d'alésages borgnes colinéaires (2) soit parallèle à toutes les autres.

9. Système comprenant :
une cartouche (1) selon la revendication 5,
un récepteur de cartouche dimensionné et formé pour s'apparier à la cartouche (1) ;
un système de manipulation de liquide capable de percer le film perçable fermant l'un quelconque de la pluralité d'alésages borgnes (2) en insérant une conduite de liquide dans l'un quelconque des alésages borgnes (2) lorsque la cartouche (1) a été appariée au récepteur de cartouche ; et
un dispositif de commande raccordé opérationnellement au système de manipulation de liquide, le dispositif de commande étant programmé pour amener le système de manipulation de liquide à :
former une cellule de liquide composite dans l'un des alésages borgnes (2) ; et
fonctionner sur la cellule de liquide composite et les réactifs de façon à réaliser le protocole biochimique prédéterminé.

10. Système selon la revendication 9, comprenant au moins l'un de ce qui suit :
- un thermocycleur,
- une unité de refroidissement,
- un module d'excitation et de détection de fluorescence optique,
- dans lequel la conduite de liquide comprend un tube capillaire,
- dans lequel la conduite de liquide comprend un module de séparation magnétique.

11. Procédé de réalisation d'un protocole biochimique comprenant :
la fourniture d'un système selon la revendication 9,
le dépôt d'un échantillon biologique dans le premier alésage borgne ;
l'insertion de la cartouche (1) dans le récepteur de cartouche ;
l'activation du dispositif de commande de sorte que le dispositif de commande amène le système de manipulation de liquide à :
former une cellule de liquide composite qui contient une solution aqueuse de l'échantillon biologique ; et
fonctionner sur la cellule de liquide composite et les réactifs de façon à réaliser le protocole biologique prédéterminé sur l'échantillon biologique.

12. Procédé selon la revendication 11, dans lequel le protocole biologique prédéterminé est conçu pour réaliser l'un quelconque parmi (a) une préparation de banque, (b) un séquençage d'acide nucléique, (c) une PCR, (d) une PCR numérique, et (e) une PCR quantitative.

13. Système comprenant une conduite, une pompe, un premier fluide, un second fluide, et un échantillon, dans lequel :
la conduite définit une ouverture haute et une ouverture basse, et comporte une surface interne disposée entre l'ouverture haute et l'ouverture basse ;
le premier fluide, l'échantillon, et le second fluide sont disposés au sein de la conduite entre l'ouverture haute et l'ouverture basse ;
le premier fluide, le second fluide et l'échantillon sont mutuellement non miscibles ;
le premier fluide est moins dense que l'échantillon et l'échantillon est moins dense que le second fluide ;
l'échantillon est disposé entre le premier fluide et le second fluide de sorte que la superficie totale de l'échantillon soit en contact fluidique avec uniquement le premier fluide, le second fluide, ou la surface interne de la conduite ;
la pompe est configurée pour appliquer une pression positive, une pression négative, ou aucune pression externe à un emplacement dans la conduite, et
une cartouche selon l'une quelconque des revendications 1 à 7 précédentes.

14. Procédé de distribution de l'échantillon depuis le système de la revendication 13 comprenant l'étape
de distribution depuis la conduite via l'ouverture basse du second fluide ; et
de distribution depuis la conduite via l'ouverture basse d'un volume de l'échantillon.

15. Procédé de stockage d'un échantillon dans un système, le procédé comprenant :
la fourniture d'une cartouche (1) selon l'une quelconque des revendications 1 à 7 précédentes, d'une conduite, d'une pompe, d'un premier fluide, d'un second fluide, et d'un échantillon, dans lequel
la cartouche (1) définit une ouverture haute et une ouverture basse, et comporte une surface interne disposée entre l'ouverture haute et l'ouverture basse ;
le premier fluide, le second fluide et l'échantillon sont mutuellement non miscibles ; le premier fluide est moins dense que l'échantillon et l'échantillon est moins dense que le second fluide ; et
la pompe est configurée pour appliquer une pression positive, une pression négative, ou aucune pression externe à un emplacement dans la conduite ; et
le chargement dans la conduite, dans n'importe quel ordre, du premier fluide, du second fluide, et de l'échantillon, de sorte que le premier fluide, l'échantillon, et le second fluide soient disposés au sein de la conduite entre l'ouverture haute et l'ouverture basse, et que l'échantillon soit disposé entre le premier fluide et le second fluide de sorte que la superficie totale de l'échantillon soit en contact fluidique uniquement avec le premier fluide, le second fluide, ou la surface interne de la conduite.
